# EUROPEAN PATENT APPLICATION

(11) **EP 0 535 621 A1**
(43) Date of publication of application: **07.04.1993**
(21) Application number: 92116701.1
(22) Date of filing: 30.09.1992
(51) Int. Cl.: C12N 1/04, C12N 9/00

(54) **Process for the stabilisation of biologically active material**

(30) Priority: 04.10.1991 AT 1985/91
(71) Applicant: BIOCHEMIE GESELLSCHAFT M.B.H., A-6250 Kundl (AT)
(72) Inventor: Knauseder, Franz, A-6322 Kirchbichl (AT); Schwald, Wolfgang, A-6800 Feldkirch (AT)
(74) Representative: Kleine Deters, Johannes

(57) **Abstract**

Process for the stabilisation of biologically active material, characterised in that a moving, cooled surface is brought into contact with a liquid containing the material at such a rate that the liquid freezes quickly and the resultant frozen material is removed from the surface.

## Description

The invention relates to a new process for the conversion of sensitive and unstable bio-technological products e.g. cell suspensions, enzymes or enzyme systems, into a stable form, and thus provides the long-term preservation of enzymatic activity and/or survival of cells e.g. sensitive to temperature, and or to shear forces.

In processes which are known from literature, attempts have been made to avoid the loss of enzymatic activity or to keep this loss as small as possible or to improve the survival rate of living organisms, by adding preservation or stabilisation agents (e.g. glycerol, DMSO, Velcorin^{R} or Zephirol^{R}).

The use of cryoprotectants such as dimethylsulfoxide (DMSO) and glycerol suggested by the prior art presents problems since DMSO and glycerol are biologically incompatible and have to be removed prior to the use of the living organisms in a biological system to prevent toxic reactions.

USP 4,897,353 discloses a method of protecting phosphofructokinase against loss of biological activity due to freezing by exposing the phosphofructokinase to an amino acid or trimethylamine-N-oxide and Zn ions.

GB Patent Application 2091534 describes a method of preserving cells comprising the step of forming a water-in-oil emulsion from an aqueous suspension of the cells and a non-toxic hydrophobic liquid (the 'oil' e.g. silicone oils and glycerides), the cells being contained in the aqueous phase of the emulsion, reducing the temperature of the emulsion to a value at which the water within the cells is frozen, and storing the emulsion at a temperature of -70° or below. In this process the hydrophobic liquids require removal.

The present invention provides a process for stabilising biologically active material e.g. cell suspensions, enzymes or enzyme systems such that their biological activity is preserved after freezing.

The invention also provides a process that will permit a biologically active material, e.g. cell suspensions, enzymes or enzyme systems to be frozen and thawed while retaining a significant amount of their biological activity.

The present invention provides in one aspect a process for the stabilisation of biologically active material characterised in that a moving cooled surface is brought into contact with a liquid containing the material at such a rate that the liquid freezes quickly and the resultant frozen material is removed from the surface.

The process according to the invention is characterised in that a dispersion, e.g. a suspension or solution e.g. in water, in particular a cell suspension or enzyme solution produced in known manner (fermentation and processing) is transformed by a very rapid freezing process into shard-like e.g. broken frozen (ice) fragments (flakes). In this process, the cell suspension or the enzyme solution is continuously or batchwise applied from a container onto a moving, cooled surface, and the biologically active material which has frozen solid after only a short time is removed with a suitable device. The moving surface may be e.g. a roller or a band conveyor. The removal of the solid may take place in such a way that the solidly frozen, biologically active material, which as a result of internal stress breaks into smaller fragments whilst still on the roller, is taken off by an ice diverter. The temperature of this surface should be at least -10°C to -40°C. Depending on the constitution of the cell suspension (viscosity of the suspension), the retention time on the cooled surface is between 5 and 100 seconds. The thickness of the solid layer is e.g. from 0.5 to 3 mm, usually 1 to 2 mm. By selecting appropriately the temperature of the submitted cell suspension or enzyme solution (room temperature or cooling of the substrate), combined with a roller temperature which corresponds to the refrigerating agent used, a drop in temperature of the substrate by 10 to 40°C may be attained.

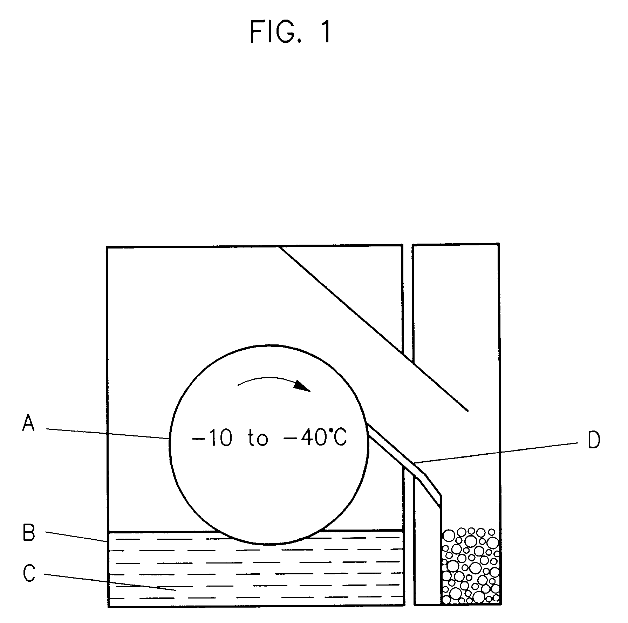

A suitable device for carrying out the process of the invention is shown in the accompanying drawing (Fig. 1) and references hereinafter in parentheses refer to the drawing.

For example the process may be effected by immersing a rotating cooled roller (A) into a container (B) filled with a cell suspension or enzyme solution (C) and removing the frozen layer with a suitable device, e.g. a knive (D) after only a short time, e.g. 20 to 100 seconds, especially 20 to 50 seconds. The rotational speed of the roller may be adjustable and is chosen in such a way to allow a rapid freezing. The rotational speed of the roller may be from e.g. 6 to 14 rpm, especially 8 to 12 rpm, usually 10 rpm. The roller may be cooled with conventional refrigerating agents, e.g. chlorofluorocarbons or fluorocarbons. The obtained dry product which is frozen under optimised conditions is preferably collected in an isolated container. Care should be taken that the roller temperature, retention time and substrate temperature are selected such that completely frozen and dry ice fragments are obtained e.g. in form of flakes. In contrast to this, in conventional laboratory ice machines, pieces of ice are produced which are still moist and therefore grip together in the supply container.

The roller may be for example of the type of a rotary evaporator used for ice freezing ( e.g. that obtained from Maja Maschinenfabrik, Kehl-Goldscheuer, Germany). Examples of cell suspensions, enzymes or enzyme systems are hydantoinase/carbamoylase, Ophiostoma specie, Ceratocystis or Leptographium.

One advantage of the process according to the invention is that due to the rapid freezing of the liquid and to the small strength of the resultant frozen layer containing the biologically active material, the ice fragments thus produced, which contain biologically active material, may be thawed very rapidly, thus avoiding activity-damaging influences such as those which occur during slow thawing of large lumps of ice. The ice fragment measures e.g. about 5 cm².

The process of the present invention is suitable for a production plant. It may be run continuously or batchwise. No additives or stabilising agents are required. The product obtained is stable and in an easily handleable form. It may be easily suspended in aqueous or buffered solutions.

The process has been tested on several cell suspensions, whereby in contrast to normal freezing processes, in a cell suspension prepared from bacterial fermentation the enzyme system hydantoinase/carbamoylase was successfully stabilised over a period of several months. The frozen ice fragments produced according to the process were stable both in storage and on transport, and could be employed without loss of enzymatic activity. Even with cell suspensions from fungal fermentation, e.g. resin-degrading cells of the genera Ophiostomales, Ceratocystis or Leptographium may be successfully converted into a form which is stable both in transport and on storage using the process according to the invention, in contrast to conventional freezing or the usage of stabilisation agents of chemical nature (e.g. DMSO). After a period of several months, the viability of the cells which are resuspended after rapid thawing was practically unchanged.

The following examples illustrate the invention without limiting its scope.

### Example 1: Stabilisation of the enzyme system hydantoinase/carbamoylase:

### 1.1 Conventional methods

Several batches for stabilising this enzyme system were produced. Besides chemical additives such as formaldehyde or benzalkonium chloride, also deep-freezing was examined in comparison to storage at 4°C. The results are given in Table 1 below:

**Table 1**

| **Activity values of the enzyme system hydantoinase/carbamoylase (expressed as R-value) in dependency of storage** | | | | |
|---|---|---|---|---|
| Product | Starting value | Enzymatic activity (R-value) | | |
| | | After 1 week | After 3 weeks | Remarks |
| batch 1 | 7.0 | 3.5 | 3.5 | ice block at -20°C |
| batch 2 | 11.0 | n.d. | 7.7 | slurry at 4°C |
| batch 3 | 6.3 | 5.0 | 3.9 | " |
| batch 4 | 6.3 | 4.6 | -+) | HCHO-addition/4°C |
| batch 5 | 11.7 | 5.0* | n.d. | Zephirol^{R} addition/4°C |
| batch 6 | 13.3 | n.d. | 3.8** | ice block at -20°C |
| n.d. = not determined -+) = not calculable | | | | |

| | | | | |
|---|---|---|---|---|
| * = after 2 weeks | | | | |
| ** = after 2 months | | | | |

The products were produced under substantially identical conditions as far as fermentation and subsequent processing are concerned.

In the case of the enzyme system hydantoinase/carbamoylase, after fermentation and downstream processing, the resulting biomass suspension is stored in an intermediate receptacle, in which the suspension is kept cool (e.g. at 4°C) and the pH is controlled (e.g. pH 7.5) until further processed.

### 1.2 Production of frozen fragments

Using the process according to the invention, the stable products described in Table 2 could be obtained in the form of frozen fragments and stored for a long period of time:

**Table 2**

| **Activity values of shard ice of the enzyme system hydantoinase/carbamoylase (expressed as R-value) after being stored at -20°C** | | | | | | |
|---|---|---|---|---|---|---|
| Product | Starting value | Enzymatic activity (R-value) after | | | | |
| | | 1 week | 3 weeks | 2 months | 3 months | 4 months |
| batch 1 | 13.4 | 12.7 | 13.4 | n.d. | n.d. | n.d. |
| batch 2 | 8.8 | n.d. | 9.3 | n.d. | n.d. | n.d. |
| batch 3 | 11.4 | n.d. | n.d. | 11.3 | 11.2 | 11.4 |
| batch 4 | 10.5 | n.d. | n.d. | 12.4 | 11.6 | 13.2 |
| batch 5 | 11.4 | n.d. | n.d. | 13.0 | 10.7 | 12.9 |
| n.d. = not determined | | | | | | |

Depending on the configuration of the ice machine (output in kg ice/h) a quantity of biomass suspension corresponding to the output of the machine e.g. 1000 kg is conveyed by a pump from the container to the reservoir of the ice machine (e.g. 27 kg/h), in which is located the cooled (e.g. -25°C) rotating roller (supplied by e.g. Maja-Maschinenfabrik, D-7640 Kehl-Goldscheuer, Germany, Type: BPL 1650), on which the suspension freezes completely within a short time (e.g. 30 seconds). Due to the internal stress occurring in the biomass-containing surface layer, cracks are formed, and the ice fragments arising therefrom can be taken off by an ice diverter. The ice-like biomass thus produced is collected in an isolated container or in a container which is pre-cooled e.g. with dry ice, and stored at -20°C.

It is thus evident that the enzymatic activity is preserved for months using this new process, in contrast to conventional methods in which activity losses of 50% and higher occur even after a relatively short time (e.g. 2 weeks).

### Example 2: Preservation of isolated biomass arising from the fermentation of Ophiostoma species

### 2.1. Conventional methods

Cells arising from the fermentation of Ophiostoma species were isolated in known manner and stabilised by mixing with additives, such as glycerol, DMSO or skimmed milk. The number of live organisms determined after storing for two weeks is listed in Table 3.

**Table 3**

| **Number of live organisms of Ophiostoma cells with and without preservatives after storing for 2 weeks at various temperatures** | | |
|---|---|---|
| Product | Number of live organisms(CFU/g) | |
| | Initial value | After 2 weeks |
| batch 1 + glycerol/-20°C | 0.5 x 10⁹ | 0.5 x 10⁹ |
| batch 1 + glycerol/4°C | 0.5 x 10⁹ | 0 |
| batch 2 + DMSO/-40°C | 2 x 10⁹ | < 10⁶ |
| batch 3 + skimmed milk/-40°C | 2 x 10⁹ | < 10⁶ n.a. = not analysed |

It can be seen that neither the stabilising agents employed nor the temperatures are suitable for maintaining the viability of the cells in the present product even for only a very short period of time.

### 2.2. Production of frozen fragments containing the cells of Ophiostoma species

Frozen fragments from a concentrated cell suspension of the species Ophiostoma were produced according to the invention and tested for their stability. The results are summarised in Table 4.

**Table 4**

| Storage time at -20°C | Number of live organisms in product (CFU/g) | |
|---|---|---|
| | batch 4 | batch 5 |
| 1 week | n.d. | 1 x 10¹⁰ |
| 2 weeks | 4 x 10⁹ | 7 x 10⁹ |
| 3 weeks | 5 x 10⁹ | 3 x 10⁹ |
| 4 weeks | 3 x 10⁹ | n.d. |
| 3 months | 2 x 10¹⁰ | n.d. |
| 4 months | 4 x 10¹⁰ | 2 x 10¹⁰ |
| n.d. = not determined | | |

The results show that with the frozen fragments produced according to the invention, there is no reduction in the number of live organisms even after a period of 4 months.

## Claims

1. A process for the stabilisation of biologically active material, characterised in that a moving cooled surface is brought into contact with a liquid containing the material at such a rate that the liquid freezes quickly and the resultant frozen material is removed from the surface.

2. A process according to claim 1, characterised in that the temperature of the cooled surface is at least -10° to -40°C.

3. A process according to claims 1 and 2, characterised in that the retention time on the cooled surface is between 5 and 100 seconds depending on the constitution of the cell suspension.

4. A process according to claims 1 to 3, characterised in that hydantoinase/carbamoylase is used as the enzyme system.

5. A process according to claims 1 to 3, characterised in that the biomass used is a fermentation mass of the Ophiostoma species.

6. A process according to claim 1 wherein the moving surface is a roller.

7. A process according to claim 1 wherein the liquid is water.
